# EUROPÄISCHE PATENTSCHRIFT

(11) **EP 1 688 107 B1**
(45) Veröffentlichungstag und Bekanntmachung des Hinweises auf die Patenterteilung: **30.07.2008**
(21) Anmeldenummer: 06001794.4
(22) Anmeldetag: 28.01.2006
(51) Int. Cl.: A61F 5/02, A61F 5/30

(54) **Rückenpelotte zur Massage und Stimulation der Rückenmuskulatur, insbesondere im Lumbosakralbereich**
Back pad for massage and stimulation of the back musculature, specially in the lumbar sacral area
Coussin d'appui pour le dos pour le massage et la stimulation de la musculature de dos, notamment de la zone lombaire sacrée

(30) Priorität: 03.02.2005 DE 102005005005
(43) Veröffentlichungstag der Anmeldung: 09.08.2006
(73) Patentinhaber: Lohmann & Rauscher GmbH & Co. KG, 56567 Neuwied (DE)
(72) Erfinder: Sandhof, Marc Christian, 56579 Rengsdorf (DE)
(74) Vertreter: Flaccus, Rolf-Dieter

(56) Entgegenhaltungen:
- DE-A1- 2 722 563
- DE-A1- 19 603 173
- DE-U1- 29 701 001
- DE-U1- 29 803 417
- US-A- 4 597 386

## Beschreibung

Die vorliegende Erfindung bezieht sich auf Rückenpelotten, die zur Massage und Stimulation der Rückenmuskulatur, insbesondere im lumbosakralen Bereich der Wirbelsäule, verwendet werden können..

Das Dokument DE-U-29701001 stellt den nächsten Stand der Technik dar.

Rückenpelotten werden in verschiedenen Formen zur Stützung und Entlastung der Wirbelsäule sowie zur Massage der Rükkenmuskulatur verwendet. Die Pelotte wird hierbei mittels einer Bandage in dem jeweiligen Bereich des Rückens fixiert, so daß während des Tragens der Pelotte am Körper ein Stützeffekt bzw. ein Massage-Effekt bewirkt wird.

In DE 101 03 545 A1 wird eine Pelotte, insbesondere für eine Rückenstützbandage, beschrieben, die auf der dem Körper zugewandten Seite zumindest eine dammartige, linienförmige Erhebung aufweist. Die dammartigen Erhebungen erstrecken sich von einem seitlichen Rand der Pelotte bis zum gegenüberliegenden Rand. An den Enden der dammartigen Erhebung ist zumindest ein erhöhter Friktionspunkt vorhanden. Die erhöhten Friktionspunkte sollen eine Friktion der medialen und lateralen Stränge der Rückenmuskulatur bewirken.

Die durch die aus DE 101 03 545 A1 bekannte Pelotte erzielte Massage- bzw. Stimulationswirkung ist jedoch nicht befriedigend. Die Ursache hierfür ist vermutlich darin zu sehen, daß nur an den seitlichen Enden der dammartigen Erhebungen Friktionspunkte vorgesehen sind. Dadurch ist der **Bereich, in dem eine lokale Massage oder Stimulation der** Rückenmuskulatur bewirkt wird, sehr beschränkt. Nachteilig ist außerdem, daß die dammartigen Erhebungen jeweils bis zum gegenüberliegenden seitlichen Rand verlaufen. Dadurch können im Bereich der Dornfortsätze unerwünschte Friktionen mit der Haut verursacht werden. Außerdem ist die MassageWirkung dieser Pelotte auf den Lendenwirbelbereich begrenzt; sie ist nicht zur Massage oder Stimulation des sakralen Bereichs geeignet.

DE 202 10 170 U1 beschreibt einen Stützkörper für den Lendenwirbelbereich, der mittels einer Bandage am Körper befestigt werden kann, und der als Rückenstütze und zur Warmhaltung für den Lendenwirbelbereich dient. Der Stützkörper hat eine tellerartige Form mit einer Symmetrieachse parallel zur Längserstreckung der Wirbelsäule. Auf der dem Körper und der Wirbelsäule zugewandten Kontaktseite ist beiderseits der Symmetrieachse jeweils ein stegförmig und in Körperlängsrichtung verlaufender Wellenberg zur Anlage an den autochthonen Rückenmuskeln ausgebildet. Zwischen den beiden Wellenbergen befindet sich ein durchgehendes Wellental zur Aufnahme der Dornfortsätze. Dieses Wellental erreicht im mittleren Bereich des Stützkörpers seine maximale Tiefe. Durch diese Ausgestaltung des Wellentals soll die Entstehung von Druck auf die Dornfortsätze vermieden werden.
Diese Anordnung von Wellenbergen und einem dazwischen liegenden Wellental ermöglicht zwar eine gute Stützwirkung, aber nur einen unbefriedigenden Massage- bzw. Stimulationseffekt, da die Wellenberge in derselben Richtung verlaufen wie die Rückenmuskulatur, d. h. im wesentlichen in der Körperlängsrichtung. Auch die aus DE 202 10 170 U1 bekannte Pelotte ist nicht zur Massage der sakralen Rückenmuskulatur geeignet; ihre Wirkung beschränkt sich auf die Lendenmuskulatur.

Aufgabe der vorliegenden Erfindung war es deshalb, die Nachteile der bekannten Rückenpelotten zu vermeiden oder abzumindern, und Rückenpelotten bereitzustellen, die eine verbesserte Massage- und Stimulationswirkung bezüglich der Rückenmuskulatur aufweisen.

Diese Aufgabe wird durch eine Rückenpelotte gemäß Anspruch 1 gelöst, sowie durch die in den Unteransprüchen beschriebenen bevorzugten Ausführungsformen.
Gemäß Anspruch 1 weist die erfindungsgemäße Rückenpelotte auf der für den Kontakt mit dem Rücken bestimmten Seite eine Vielzahl von wulstförmigen länglichen Erhebungen auf, die zu beiden Seiten einer Längsachse X der Rückenpelotte und in einem Abstand zu dieser Achse angeordnet sind, vorzugsweise symmetrisch zu dieser Achse, und die zum seitlichen Rand der Rückenpelotte hin verlaufen. Der Verlauf der Achse X entspricht dem Verlauf der Wirbelsäule in Längsrichtung. Die Rückenpelotte ist aus einem elastischen Kunststoffmaterial hergestellt. Ferner besitzen zumindest einige der länglichen Erhebungen in ihrem Längsverlauf knotenartigen Verdickungen oder Höcker, so daß sich ein wellenförmiges Höhenprofil mit zusätzlichen Friktionspunkten ergibt.

Die Pelotte kann, wie üblich, mittels einer Rückenbandage an der zu behandelnden Region des Rückens befestigt werden. Durch die wulstförmigen länglichen Erhebungen, die von der Längsachse X (und ein einem Abstand zu dieser) zu den seitlichen Rändern verlaufen, wird während des Tragens der Pelotte ein Massage-Effekt auf die Rückenmuskulatur ausgeübt. Zusätzlich werden dabei die neuromuskulären Rezeptoren positiv stimuliert. Von besonderer Bedeutung ist hierbei, daß die länglichen Erhebungen - aufgrund ihrer Anordnungsweise auf der Pelotte - im wesentlichen quer oder schräg zur Rückenmuskulatur verlaufen. Das heißt, daß durch die Erhebungen eine Querfriktion der dorsalen Rückenmuskulatur (M. erector spinae) in dem unterhalb der Pelotte liegenden Bereich des Rückens bewirkt wird.

Die erfindungsgemäße Pelotte ist aufgrund der vorstehend beschriebenen Anordnung von wulstförmigen Erhebungen und Noppen speziell an die anatomischen Verhältnisse der Rükkenmuskulatur angepaßt und eignet sich deshalb vorteilhaft zur therapeutischen Behandlung von Krankheiten und Symptomen, die den Lumbal- oder Lumbosakralbereich betreffen. Bekanntlich vereinen sich die Sehnen der verschiedenen Rükkenstrecker (Mm. multifidi) im Bereich des Os sacrum und im cranialen Bereich des Os coccygis in einer gemeinsamen Aponeurose. Durch die starken Arbeitskräfte der Rückenmuskulatur entstehen an diesem relativ kleinen Ansatzpunkt beträchtliche Zugkräfte. Infolgedessen können bei Überbeanspruchung insbesondere im Ansatzbereich der Sehnen pathologische Veränderungen verursacht werden (Ansatz-Tendopathologien). Die durch die wulstförmigen Erhebungen bewirkte Querfriktion ermöglicht eine optimale Massage und Stimulation dieses Bereichs der Rückenmuskulatur und damit einen äußerst günstigen Therapie-Effekt.

Außerdem kommt es während des Tragens der Pelotte zu einer leichten Erwärmung der Muskulatur, wodurch zusätzlich eine Entspannung der betroffenen Muskulatur bewirkt wird.

Des weiteren ist es von Vorteil, daß die wulstförmigen Erhebungen einen Abstand zur Längsachse X einhalten, so daß sie während des Tragens keinen unerwünschten Druck auf die Dornfortsätze ausüben können.

Die Verwendung eines elastischen Kunststoffmaterials, vorzugsweise eines Kunststoffschaums, trägt zusätzlich zur Erzielung einer guten Massagewirkung bei; zudem werden dadurch ein leichteres Gewicht, eine saubere und hautfreundliche Oberfläche sowie eine ästhetisch ansprechende Gestaltung ermöglicht. Durch die wärmeisolierenden Eigenschaften des Kunststoffschaums wird das Entstehen einer lokalen Erwärmung im Applikationsbereich begünstigt.

Aufgrund der Verwendung eines elastischen Kunststoffmaterials als Basismaterial ist die Pelotte außerdem sehr biegsam und kann sich sowohl in Längs- als auch in Querrichtung dem anatomischen Verlauf des Rückens anpassen.

Geeignete elastische Kunststoff-Materialien sind dem Fachmann bekannt; sie können insbesondere aus der PolyethylenSchaum, Silikon-Schaum oder Silikon (z. B. Silikonkautschuk, Siloxan-Elastomere), Schaumstoffe auf Basis von Natur- oder Synthesekautschuk und Polyurethan-Weichschaum umfassenden Gruppe (z. B. Schaumgummi, Moosgummi) ausgewählt sein. Polyurethan-Weichschaum ist aufgrund seiner vorteilhaften Materialeigenschaften (geringes Gewicht, saubere Oberflächen, hautfreundlich, Elastizität, Wärmeisolation, nicht allergie-auslösend) besonders bevorzugt. Als nicht geschäumte Kunststoff-Materialien kommen beispielsweise weichelastische Synthesekautschuke in Betracht. Vorzugsweise liegt die Shore-A-Härte des verwendeten elastischen Kunststoffs oder Kunststoffschaums im Bereich von 10 bis 45, besonders bevorzugt im Bereich von 15 bis 30.

Gemäß einer bevorzugten Ausführungsform der Erfindung ist vorgesehen, daß die wulstförmigen länglichen Erhebungen beiderseits der Achse X und in einem solchen Abstand zur Achse X angeordnet sind, daß längs dieser Achse ein streifenförmiger Bereich mit einer Breite von mindestens 1,5 cm, vorzugsweise von mindestens 2 cm, verbleibt, der keine Erhebungen aufweist. Dadurch werden unerwünschte Friktionen im Bereich der Dornfortsätze noch besser vermieden.

Die Anzahl der länglichen Erhebungen beträgt vorzugsweise 4 bis 20, stärker bevorzugt 6 bis 12. Hinsichtlich der geometrischen Anordnung wird bevorzugt, daß die auf einer jeden Seite der Achse X vorhandenen länglichen Erhebungen im wesentlichen parallel zueinander verlaufen. Vorzugsweise beträgt der Abstand zwischen den parallel verlaufenden Erhebungen 1,5 bis 5 cm, insbesondere 2 bis 3 cm, bezogen auf deren Längsachse. Der zwischen zwei benachbarten, parallel verlaufenden Erhebungen vorhandene ebene Bereich hat vorzugsweise eine Breite von 2 bis 20 mm, insbesondere 0,5 bis 1 cm.

Das Querschnittsprofil der länglichen Erhebungen kann vorzugsweise halbrund, breit-oval oder unregelmäßig rund geformt sein.

Die Länge der einzelnen länglichen Erhebungen hängt unter anderem von dem zu behandelnden Bereich des Rückens und von der Gesamtfläche der Pelotte ab. Vorzugsweise haben die länglichen Erhebungen eine Länge im Bereich von 1 bis 15 cm, insbesondere im Bereich von 2 bis 10 cm.
Weiterhin wird bevorzugt, daß zumindest zwei der auf einer Pelotte vorhandenen länglichen Erhebungen eine unterschiedliche Länge aufweisen. Auch in diesem Fall wird eine symmetrische Anordnung bezüglich der Längsachse X bevorzugt.
Auf diese Weise kann die Pelotte optimal an den Bereich des Rückens, für dessen Behandlung sie bestimmt ist, angepaßt werden.

Eine weitere, besonders bevorzugte Ausführungsform sieht vor, daß die länglichen Erhebungen V-förmig oder Λ-förmig (d. h. umgekehrt V-förmig) angeordnet sind. Dabei wird bevorzugt, daß der durch die v-förmige oder Λ-förmige Anordnung gebildete Winkel α 90° bis 175°, vorzugsweise 120° bis 160°, beträgt.
Aufgrund der V-förmigen oder Λ-förmigen Anordnung wird bewirkt, daß die wulstförmigen Erhebungen quer bzw. schräg auf der Rückenmuskulatur, die im wesentlichen in Längsrichtung verläuft, zu liegen kommen. Dadurch wird eine besonders günstige Massage- und Stimulationswirkung erzielt.

Die Höhe der länglichen Erhebungen liegt vorzugsweise im Bereich von 2-20 mm, insbesondere im Bereich von 2-5 mm (jeweils bezogen auf die flache Oberfläche in der unmittelbaren Umgebung der Erhebungen). Nach einer besonders bevorzugten Ausführungsform ist vorgesehen, daß die Höhe der länglichen Erhebungen, oder zumindest einiger dieser Erhebungen, zum seitlichen Rand der Pelotte hin abnimmt. Das heißt, die Längswülste haben ihre größte Höhe im Anfangsbereich nahe der Längsachse X, und sie werden zum Außenrand hin zunehmend flacher.

Weiterhin ist nach einer besonders bevorzugten Ausführungsform vorgesehen daß die länglichen Erhebungen, oder zumindest einige dieser Erhebungen, in ihrem Längsverlauf mit knotenartigen Verdickungen oder Höckern versehen sind, so daß sich ein wellenförmiges Höhenprofil ergibt. Durch diese Verdickungen werden zusätzliche, besonders exponierte Friktionspunkte gebildet, welche den Massage- und Stimulationseffekt verstärken.

Die vorliegende Erfindung bezieht sich insbesondere auf eine Rückenpelotte der vorstehend beschriebenen Art, die als Lumbo-Sakral-Rückenpelotte ausgebildet ist und zu diesem Zweck einen ersten Flächenbereich, in welchem die genannten wulstförmigen länglichen Erhebungen angebracht sind, sowie einen zweiten Flächenbereich, der mit einer Vielzahl von erhabenen Noppen ausgestattet ist, aufweist. Die wulstförmigen länglichen Erhebungen des ersten Flächenbereiches bewirken, wie vorstehend beschrieben, eine Massage und Stimulation der lumbalen Rückenmuskulatur sowie eine Stimulation der in diesem Bereich befindlichen neuromuskulären Rezeptoren (Propriorezeption).

Durch die Noppen des zweiten Flächenbereiches wird zusätzlich eine Massage und Stimulation der im Sakralbereich befindlichen Muskulatur und neuromuskulären Rezeptoren (Propriorezeption) ermöglicht. Da die Sehnen (der Rückenstrekker) im unteren Bereich (Sakralbereich) sehr schmal sind, wird hier die Querfriktion der Sehnen und Muskulatur erfindungsgemäß durch die genannten Noppen bewirkt. Die dadurch erzielte therapeutische Wirkung wird zusätzlich durch den propriorezeptiven Reiz, der durch die Noppen der Pelotte ausgeübt wird, unterstützt. Diese Druckreize im Sakralbereich, die sich auf die Haut und die darunterliegenden Strukturen auswirken, stimulieren bestimmte Rezeptoren, die auf Druck, Zug, Spannung und Lageveränderung reagieren, positiv. Entsprechendes gilt im Hinblick auf die oben erwähnten wulstförmigen Erhebungen. Dies bedeutet, daß die Pelotte sowohl muskulär, ligamentär und propriorezeptiv wirkt.

Vorzugsweise sind die genannten Noppen im Grundriß kreisrund, ellipsenförmig drei-, vier- oder vieleckig, oder unregelmäßig rund; ihr Durchmesser beträgt vorzugsweise 0,5 cm bis 2 cm, besonders bevorzugt 1 cm bis 1,5 cm, und ihre Höhe liegt vorzugsweise im Bereich von 0,2 cm bis 1 cm. Die Anzahl der Noppen kann in einem weiten Bereich variiert werden; vorzugsweise sind 4 bis 30, besonders bevorzugt 8 bis 20, solcher Noppen vorhanden. Ferner wird bevorzugt, daß die Noppen bezüglich der Längsachse X symmetrisch angeordnet sind. Der Seitenabstand zwischen den einzelnen benachbarten Noppen entspricht vorzugsweise mindestens dem halben Durchmesser dieser Noppen.

Des weiteren ist vorgesehen, daß mindestens zwei Gruppen von Noppen vorhanden sind, die sich bezüglich ihres Durchmessers unterscheiden, wobei jede Gruppe mindestens zwei Noppen umfaßt. Dabei wird bevorzugt, daß die im caudalen Bereich gelegenen Noppen einen kleineren Durchmesser aufweisen als die im cranialen Bereich gelegenen Noppen; letzter können - abweichend von der kreisrunden Form - auch länglich geformt sein und zum Außenrand der Pelotte oder/und zur mittleren Längsachse hin verlängert sein.

Vorzugsweise weist die genannte Lumbo-Sakral-Pelotte im Grundriß im wesentlichen die Form eines gleichschenkligen Dreiecks oder gleichschenkligen Trapezes auf. Dabei ist es vorteilhaft, wenn die Ecken des Dreieckes oder Trapezes abgerundet sind; des weiteren können die Seiten konkav oder konvex gerundet sein. Beim Tragen der Pelotte am Körper ist eine Spitze des Dreiecks oder Trapezes in Körperlängsrichtung nach unten (in caudaler Richtung) ausgerichtet; dieser nach unten gerichtete Flächenabschnitt des Dreiecks oder Trapezes enthält die genannten Noppen und ist, wie erwähnt, zur Massage und Stimulation des Sakralbereichs bestimmt.

Der im wesentliche flache Grundkörper der Pelotte, an dem die wulstförmigen Erhebungen bzw. Noppen ausgebildet sind, hat vorzugsweise eine Dicke im Bereich von 0,5 bis 1,5 cm. Gemäß einer besonders bevorzugten Ausführungsform bildet die Pelotte keine vollkommen gerade Ebene. Insbesondere können die seitlichen Bereiche gemäß der anatomischen Form des Rückens aus der Ebene heraus nach oben gebogen sein. Des weiteren kann bei einer Lumbo-Sakral-Pelotte der genannte zweite Flächenbereich, welcher der Massage bzw. Stimulation der Sakralmuskulatur dient, nach oben (d. h. in ventraler Richtung) gebogen sein. Dadurch wird der auf die Sakralmuskulatur ausgeübte Druck erhöht und die Massage-und Stimulationswirkung in diesem Bereich verbessert.

Die Herstellung der erfindungsgemäßen Pelotten kann unter Verwendung der angegebenen Materialien auf dem Fachmann bekannte Weise erfolgen. Gemäß einer besonders bevorzugten Ausführungsform ist die Rückenpelotte einstückig hergestellt. Dies kann beispielsweise mittels einer Negativ-Gußform oder Spritzform erfolgen, in welcher entsprechende vertiefungen für die Ausbildung der länglichen Erhebungen bzw. der Noppen angebracht sind. Auf diese Weise ist eine einfache und kostengünstige Herstellung größerer Stückzahlen möglich.

Um eine sichere Befestigung der Pelotte am Rücken eines Patienten mittels einer Rückenbandage zu ermöglichen, ist es vorteilhaft, an der Unterseite, welche der für den Kontakt mit dem Rücken bestimmten Seite gegenüber liegt, ein oder mehrere Befestigungsmittel anzubringen. Diese können insbesondere aus der Gruppe der Klettverschlüsse, Druckknöpfe, Schlaufen, Ösen und Haken ausgewählt sein. Geeignete Bandagen zur Befestigung der Pelotte am Rücken einer Person sind dem Fachmann bekannt. Ferner ist alternativ vorgesehen, die erfindungsgemäße Pelotte an der Rückenlehne eines Sitzmöbels oder eines Fahrzeugsitzes anzubringen oder dort einzubauen, so daß auf diese Weise eine Massage der Rückenmuskulatur einer sitzenden Person ermöglicht wird.

Des weiteren ist nach einer bevorzugten Ausführungsform vorgesehen, daß die Rückenpelotte von einer Hülle oder Tasche umgeben ist, welche zumindest die Oberfläche der für den Kontakt mit dem Rücken bestimmten Seite bedeckt. Im allgemeinen hat diese Hülle oder Tasche dieselbe Grundrißform wie die Pelotte, für deren Aufnahme sie bestimmt ist. Beispielsweise kann eine solche Hülle aus zwei Stoffbahnen, die entsprechend der geometrischen Form der Pelotte zugeschnitten und an ihren Rändern miteinander vernäht wurden, gebildet sein.

Die Hülle oder Tasche ist vorzugsweise aus einem textilen Material, beispielsweise Natur- oder Kunstfasern, hergestellt, und sie ist vorzugsweise mit einer Aussparung oder Öffnung versehen, durch welche das genannte Befestigungsmittel (z. B. Klettstreifen) von außen zugänglich ist, wenn die Pelotte in die Tasche eingesteckt ist. Dadurch ist es möglich, die in ihre Hülle eingesteckte Pelotte mittels des an der Unterseite der Pelotte vorhandenen Befestigungsmittels an einer dazugehörigen Bandage zu befestigen. Die genannte Öffnung oder Aussparung ermöglicht zudem ein Herausnehmen und Wiedereinsetzen der Pelotte in die Hülle, beispielsweise zur Reinigung der Hülle. Die Verwendung einer Hülle oder Tasche ist einerseits aus hygienischen Gründen empfehlenswert, andererseits wird dadurch auch der Tragekomfort erhöht.

Die Erfindung wird nachfolgend anhand von Zeichnungen, die sich auf eine besonders bevorzugte Ausführungsform der erfindungsgemäßen Pelotte beziehen, veranschaulicht. Die Bezugszeichen haben in allen Zeichnungen im wesentlichen dieselbe Bedeutung.

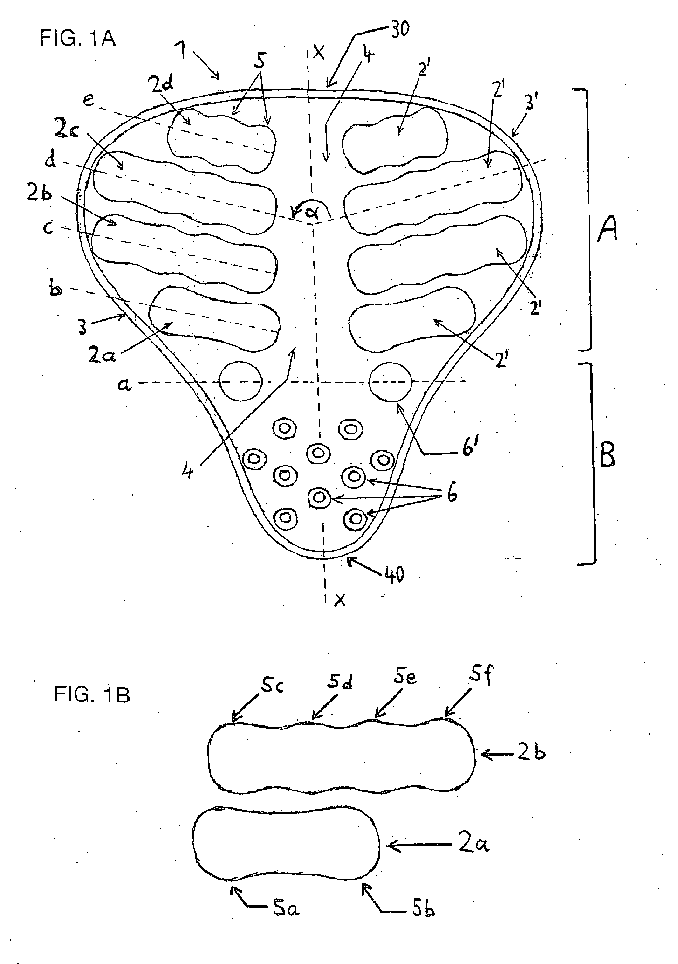

FIG. 1A zeigt die Rückenpelotte (1) in der Draufsicht. In diesem Fall handelt es sich um die bevorzugte Ausführungsform einer Lumbo-Sakral-Rückenpelotte. Die Pelotte weist eine Vielzahl von wulstförmigen länglichen Erhebungen (2a, 2b, 2c, 2d, 2') auf, die zu beiden Seiten der Längsachse X der Rückenpelotte und in einem Abstand zu dieser Achse symmetrisch angeordnet sind. Die wulstförmigen länglichen Erhebungen erstrecken sich zum seitlichen Rand (3, 3') hin. **Der Verlauf der Achse X entspricht im wesentlichen dem Ver**lauf der Wirbelsäule in ihrer Längsrichtung. Das vordere (craniale) Ende der Pelotte ist mit (30), das hintere (caudale) Ende ist mit (40) bezeichnet.

Die länglichen Erhebungen (2a, 2b, 2c, 2d, 2') sind in einem solchen Abstand zur Achse X angeordnet, daß längs dieser Achse ein streifenförmiger Bereich (4) mit einer Breite von 2,5 bis 3 cm verbleibt, der keine Erhebungen aufweist, sondern flach ist.

Es sind insgesamt acht längliche Erhebungen vorhanden, von denen jeweils vier auf jeder Seite der Achse X angeordnet sind. Die auf einer jeden Seite der Achse X angeordneten länglichen Erhebungen verlaufen im wesentlichen parallel zueinander. Die Länge der Erhebungen beträgt 5 cm (2a), 7 cm (2b, 2c), bzw. 4 cm (2d). Ihre Breite beträgt 1,5 bis 2 cm. Die zwischen den Erhebungen vorhandenen flachen Bereiche haben eine Breite von ca. 3-7 mm. Der Abstand zwischen zwei benachbarten Erhebungen beträgt ca. 2,5 cm (bezogen auf die Schnittlinien b, c, d, e).

Die länglichen Erhebungen (2a, 2b, 2c, 2d, 2') sind V-förmig angeordnet, wobei der dadurch gebildete Winkel α ca. 135° beträgt.
Ferner sind die länglichen Erhebungen mit knotigen Verdickungen oder Höckern (5) versehen, die in der Draufsicht als Ausbuchtungen erkennbar sind.

Die Lumbo-Sakral-Rückenpelotte hat die Form eines gleichschenkligen Dreiecks mit gerundeten Ecken, und wobei die craniale Dreiecksseite nach außen gebogen und die beiden anderen Dreiecksseiten nach innen gebogen sind.
Die gesamte Länge der Pelotte, gemessen entlang der Achse X, beträgt ca. 19 cm.
Bei der Herstellung dieser Pelotte wurde ein expandiertes Polyurethansystem verwendet (Polyurethanschaum mit einer Shore-A-Härte von 22 ± 3); die Herstellung erfolgte mittels einer Spritzform.

Die Lumbo-Sakral-Rückenpelotte weist einen ersten Flächenbereich (A; cranial), in welchem die genannten wulstförmigen länglichen Erhebungen (2a, 2b, 2c, 2d, 2') angebracht sind, sowie einen zweiten Flächenbereich (B; caudal) auf, der mit zwölf erhabenen Noppen (6, 6') ausgestattet ist. Die Noppen sind annähernd kreisrund mit einem Durchmesser von ca. 7 mm bzw. 15 mm. Die im oberen Bereich des zweiten Flächenbereichs B bzw. im Übergangsbereich zwischen den beiden Flächenbereichen A und B liegenden Noppen (6') weisen bevorzugt einen größeren Durchmesser auf als die weiter unten (kaudal) liegenden Noppen (6). Des weiteren können die Noppen (6') von der Kreisform abweichen und zum Rand hin oder/und zur Mittelachse X hin verlängert sein (z. B. längsoval oder ellipsenförmig).
Der erste Flächenbereich (A) dient der Massage und Stimulation des Lumbalbereichs, der zweite Flächenbereich (B) dient der Massage und Stimulation des Sakralbereichs.

Falls die erfindungsgemäße Pelotte als Lumbal-Pelotte gestaltet ist und nur den Flächenbereich A, nicht aber den Flächenbereich B umfaßt, verläuft ihr Außenrand (3) im unteren Bereich beispielsweise entlang der gestrichelten Linie (a), wobei auch dieser untere Rand - wie beschrieben - gebogen oder ausgebuchtet sein kann.

FIG. 1B zeigt, ebenfalls in Draufsicht, zwei der in FIG. 1A abgebildeten länglichen Erhebungen (2a, 2b) mit Verdickungen (5a - 5 f), die als seitliche Ausbuchtungen erkennbar sind.

Die FIG. 2A, 2B, 2C, 2D und 2E zeigen Schnittdarstellungen entlang der in FIG. 1A eingezeichneten Linien (a) bis (e). Der flache Grundkörper der Pelotte (ohne Noppen oder Erhebungen) hat eine Dicke von ca. 0,5 cm.

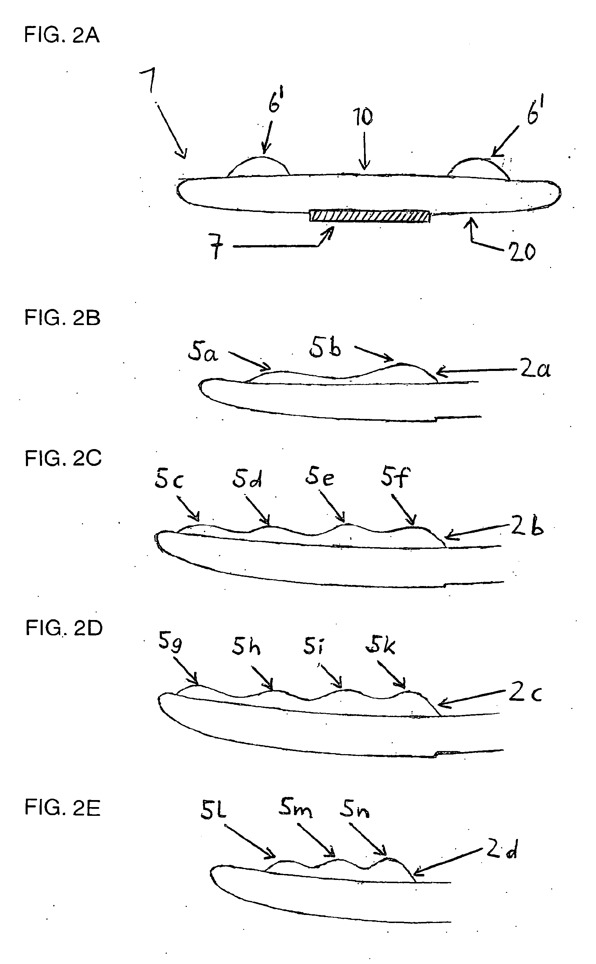

FIG. 2A zeigt zwei im Bereich der Linie (a) befindliche Noppen (6'), deren Höhe ca. 5 mm beträgt (gemessen von der flachen Oberfläche, welche die Noppen umgibt). Die für den Kontakt mit dem Rücken bestimmte Seite ist mit (10), die ihr gegenüberliegende Rückseite ist mit (20) bezeichnet. An der Rückseite ist mittig ein Klettverschlußstreifen (7) als Befestigungsmittel angebracht.

FIG. 2B zeigt eine längliche Erhebung (2a) mit daran ausgebildeten knotigen Verdickungen oder Höckern (5a, 5b), deren Höhe (gemessen von der flachen Oberfläche, welche die Erhebung umgibt) ca. 3mm (5a) bzw. ca. 5 mm (5b) beträgt.

FIG. 2C zeigt eine längliche Erhebung (2b) mit daran ausgebildeten knotigen Verdickungen oder Höckern (5c - 5f), deren Höhe 3 mm (5c), ca. 4 mm (5d), ca. 6 mm (5e) bzw. ca. 5,5 mm (5f) beträgt.

FIG. 2D zeigt eine längliche Erhebung (2c) mit daran ausgebildeten knotigen Verdickungen oder Höckern (5g - 5k), deren Höhe ca. 3,3 mm (5g), ca. 4,5 mm (5h), ca. 6,4 mm (5i) bzw. ca. 6,5 mm (5k) beträgt.

FIG. 2E zeigt eine längliche Erhebung (2e) mit daran ausgebildeten knotigen Verdickungen oder Höckern (51 - 5n), deren Höhe ca. 3,6 mm (51), ca. 5 mm (5m) bzw. ca. 6,2 mm (5n) beträgt.

Wie aus FIG. 2B - 2E ersichtlich, haben die Erhebungen (2) ein wellenartiges Höhenprofil, wobei die Höhe dieser Erhebungen zum Außenrand der Pelotte hin abnimmt. Dadurch wird erreicht, daß die Pelotte, wenn sie am Rücken befestigt ist, an ihren seitlichen Rändern einen flachen Übergang zu den umgebenden Körperregionen des Patienten aufweist, so daß die Pelotte weitgehend unauffällig unter der Kleidung verborgen werden kann, und keine hervorstehenden Kanten oder Konturen erkennbar sind.

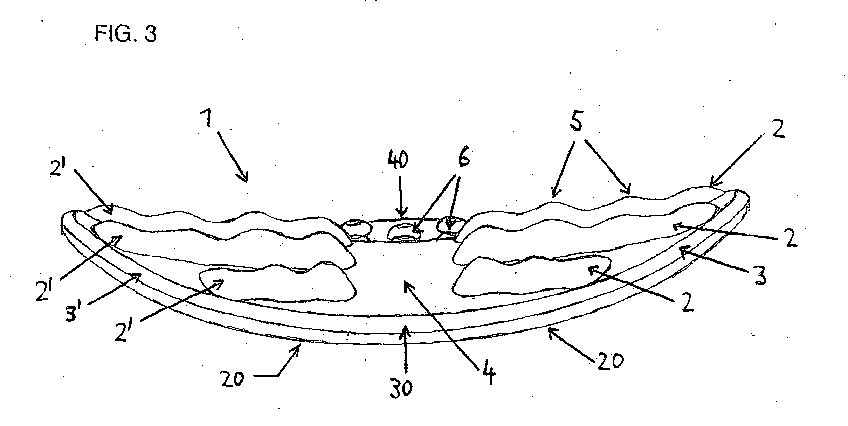

FIG. 3 zeigt die in FIG. 1 abgebildete Pelotte von vorne (Frontansicht). Wie erkennbar, ist die Pelotte gemäß der anatomischen Form eines menschlichen Rückens leicht gekrümmt. Ihre Breite beträgt ca. 17,5 cm (gemessen an der breitesten Stelle).

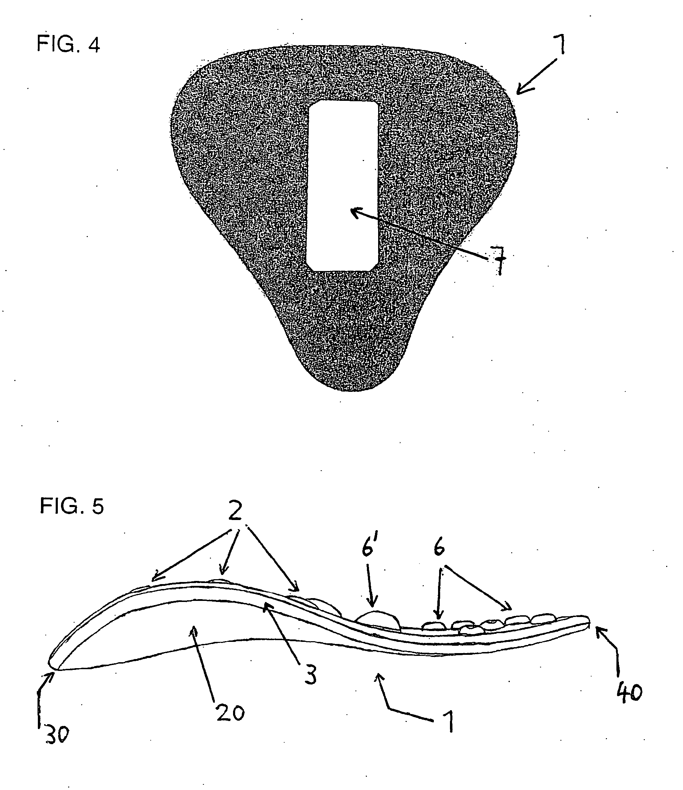

FIG. 4 zeigt die Unterseite (20) in FIG. 1 abgebildeten Pelotte, in Draufsicht. In der Mitte der Fläche ist ein Klettverschlußstreifen (7) angebracht (Größe ca. 3 x 10 cm), der in eine entsprechende Vertiefung auf der Unterseite eingelassen ist.

FIG. 5 zeigt die in FIG. 1 abgebildete Pelotte in einer Seitenansicht. Hierbei ist die den anatomischen Verhältnissen entsprechende Formgebung deutlich zu erkennen. Durch die kurvenartige Krümmung auch in Längsrichtung, insbesondere durch die leichte Aufwärtskrümmung des caudal gelegenen Bereichs (40) wird die Massage- und Stimulationswirkung während des Tragens erheblich verbessert.

Die erfindungsgemäßen Rückenpelotten können vorteilhaft zur therapeutischen und auch prophylaktischen Behandlung von Krankheiten und Symptomen des Rückens und der Rückenmuskulatur verwendet werden. Hierzu wird eine erfindungsgemäße Pelotte mittels einer geeigneten Bandage über dem zu behandelnden Abschnitt des Rückens eines Patienten befestigt, so daß die wulstförmigen Erhebungen und die Noppen eine Massage und Stimulation der Rückenmuskulatur bewirken.

Insbesondere kann eine erfindungsgemäße Rückenpelotte für folgende Behandlungszwecke verwendet werden:
- zur Massage und Stimulation der Rückenmuskulatur, insbesondere im lumbosakralen Bereich der Wirbelsäule; oder
- zur Hyperämisierung der Rückenmuskulatur, insbesondere im lumbosakralen Bereich der Wirbelsäule; oder
- zur Behandlung von Muskelverspannungen und zur Schmerzlinderung im Bereich der Rückenmuskulatur, insbesondere im lumbosakralen Bereich der Wirbelsäule; oder
- zur Behandlung von muskulärer Insuffizienz im Bereich der Rückenmuskulatur, insbesondere im lumbosakralen Bereich der Wirbelsäule; oder
- zur Stützung der Wirbelsäule, insbesondere im lumbosakralen Bereich; oder
- zur Behandlung von Haltungsfehlern und Haltungsstörungen, insbesondere im lumbosakralen Bereich; oder
- zur Behandlung von Durchblutungsstörungen oder zur Förderung der Durchblutung im Bereich der Rückenmuskulatur, insbesondere im lumbosakralen Bereich.

## Patentansprüche

1. Rückenpelotte (1) zur Massage und Stimulation der Rückenmuskulatur, die aus einem elastischen Kunststoffmaterial hergestellt ist und die auf der für den Kontakt mit dem Rücken bestimmten Seite eine Vielzahl von wulstförmigen länglichen Erhebungen (2a, 2b, 2c, 2d, 2') aufweist, die zu beiden Seiten einer Längsachse(x)der Rückenpelotte und in einem Abstand zu dieser Achse angeordnet sind, vorzugsweise symmetrisch, und die zum seitlichen Rand (3, 3') der Rükkenpelotte hin verlaufen, wobei der Verlauf der Achse(X)dem Verlauf der Wirbelsäule in Längsrichtung entspricht, **dadurch gekennzeichnet, daß** zumindest einige der länglichen Erhebungen (2a, 2b, 2c, 2d, 2') in ihrem Längsverlauf mit knotenartigen Verdickungen oder Höckern (5) versehen sind, so daß sich ein wellenförmiges Höhenprofil ergibt.

2. Rückenpelotte nach Anspruch 1, **dadurch gekennzeichnet, daß** die länglichen Erhebungen (2a, 2b, 2c, 2d, 2') in einem solchen Abstand zur Achse X angeordnet sind, daß längs dieser Achse ein streifenförmiger Bereich (4) mit einer Breite von mindestens 1,5 cm, vorzugsweise von mindestens 2 cm, verbleibt, der keine Erhebungen aufweist.

3. Rückenpelotte nach Anspruch 2, **dadurch gekennzeichnet, daß** die Breite des streifenförmigen Bereichs (4) 1,5 bis 10 cm, vorzugsweise 2 bis 5 cm, beträgt.

4. Rückenpelotte nach einem der Ansprüche 1 bis 3, **dadurch gekennzeichnet, daß** die Anzahl der länglichen Erhebungen (2a, 2b, 2c, 2d, 2') 4 bis 20, vorzugsweise 6 bis 12, beträgt, und wobei bevorzugt wird, daß die auf einer jeden Seite der Achse X angeordneten länglichen Erhebungen parallel zueinander verlaufen.

5. Rückenpelotte nach einem der vorangehenden Ansprüche, **dadurch gekennzeichnet, daß** die länglichen Erhebungen (2a, 2b, 2c, 2d, 2') eine Länge im Bereich von 1 bis 15 cm, vorzugsweise 2 bis 20 cm, aufweisen.

6. Rückenpelotte nach einem der vorangehenden Ansprüche, **dadurch gekennzeichnet, daß** die länglichen Erhebungen (2a, 2b, 2c, 2d, 2') eine Breite im Bereich von 0,5 bis 3 cm, vorzugsweise 1 bis 2 cm aufweisen.

7. Rückenpelotte nach einem der vorangehenden Ansprüche, **dadurch gekennzeichnet, daß** zumindest zwei der länglichen Erhebungen (2a, 2b, 2c, 2d, 2') eine unterschiedliche Länge aufweisen.

8. Rückenpelotte nach einem der vorangehenden Ansprüche, **dadurch gekennzeichnet, daß** die länglichen Erhebungen (2a, 2b, 2c, 2d, 2') V-förmig oder Λ-förmig angeordnet sind, wobei bevorzugt wird, daß der dadurch gebildete Winkel α 90° bis 175°, vorzugsweise 120° bis 160°, beträgt.

9. Rückenpelotte nach einem der vorangehenden Ansprüche, **dadurch gekennzeichnet, daß** die länglichen Erhebungen (2a, 2b, 2c, 2d, 2') eine Höhe von 2-20 mm, vorzugsweise 2-5 mm, aufweisen.

10. Rückenpelotte nach einem der vorangehenden Ansprüche, **dadurch gekennzeichnet, daß** die Höhe der länglichen Erhebungen (2a, 2b, 2c, 2d, 2'), oder zumindest einiger dieser Erhebungen, zum seitlichen Rand (3, 3') hin abnimmt.

11. Rückenpelotte nach einem der vorangehenden Ansprüche, **dadurch gekennzeichnet, daß** sie als Lumbo-Sakral-Rückenpelotte ausgebildet ist und einen ersten Flächenbereich (A), in welchem die genannten wulstförmigen länglichen Erhebungen (2a, 2b, 2c, 2d, 2') angebracht sind, sowie einen zweiten Flächenbereich (B), der mit einer Vielzahl von erhabenen Noppen (6, 6') ausgestattet ist, aufweist.

12. Rückenpelotte nach Anspruch 11, **dadurch gekennzeichnet, daß** die Noppen (6, 6') im Grundriß kreisrund, ellipsenförmig, drei-, vier- oder vieleckig, oder unregelmäßig rund sind, wobei bevorzugt wird, daß der Durchmesser 0,5 cm bis 2 cm, vorzugsweise 1 cm bis 1,5 cm beträgt.

13. Rückenpelotte nach Anspruch 11 oder 12, **dadurch gekennzeichnet, daß** die Noppen (6, 6') eine Höhe von 0,2 cm bis 1 cm aufweisen.

14. Rückenpelotte nach einem der Ansprüche 11 bis 13, **dadurch gekennzeichnet, daß** ihr Grundriß im wesentlichen die Form eines gleichschenkligen Dreiecks oder gleichschenkligen Trapezes hat, vorzugsweise mit abgerundeten Ecken o-der/und konkav oder konvex gerundeten Seiten.

15. Rückenpelotte nach einem der vorangehenden Ansprüche, **dadurch gekennzeichnet, daß** das Kunststoffmaterial ein Kunststoffschaum ist, vorzugsweise ausgewählt aus der Polyethylen-Schaum, Silikon-Schaum, Schaumstoffe auf Basis von Natur- oder Synthesekautschuk und Polyurethan-Weichschaum umfassenden Gruppe, wobei Polyurethan-Weichschaum besonders bevorzugt ist.

16. Rückenpelotte nach einem der vorangehenden Ansprüche, **dadurch gekennzeichnet, daß** sie einstückig hergestellt ist.

17. Rückenpelotte nach einem der vorangehenden Ansprüche, **dadurch gekennzeichnet, daß** sie auf ihrer Unterseite (20), welche der für den Kontakt mit dem Rücken bestimmten Seite (10) gegenüber liegt, ein oder mehrere Befestigungsmittel (7) aufweist, vorzugsweise ausgewählt aus der Gruppe der Klettverschlüsse, Druckknöpfe, Schlaufen, Ösen und Haken.

18. Rückenpelotte nach einem der vorangehenden Ansprüche, **dadurch gekennzeichnet, daß** sie von einer Hülle oder Tasche umgeben ist, welche zumindest die Oberfläche der für den Kontakt mit dem Rücken bestimmten Seite bedeckt.

## Claims

1. Back pad (1) for massage and stimulation of the back musculature, which back pad (1) is made of an elastic plastics material and which, on the side intended to be in contact with the back, has a plurality of bulge-like elongate elevations (2a, 2b, 2c, 2d, 2') which are arranged on both sides of a longitudinal axis (X) of the back pad and at a distance from said axis, preferably symmetrically, and extend towards the lateral edge (3, 3') of the back pad, with the course of the axis (X) corresponding to the course of the spinal column in longitudinal direction, **characterised in that** at least some of the elongate elevations (2a, 2b, 2c, 2d, 2') are provided along their lengthwise course with knot-like thickenings or humps (5), resulting in an undulating height profile.

2. Back pad according to claim 1, **characterised in that** the elongate elevations (2a, 2b, 2c, 2d, 2') are arranged at such a distance from the axis X that along said axis a strip-shaped region (4), having a width of at least 1.5 cm, preferably at least 2 cm, remains which does not have any elevations.

3. Back pad according to claim 2, **characterised in that** the width of the strip-shaped regions (4) is 1.5 to 10 cm, preferably 2 to 5 cm.

4. Back pad according to any one of claims 1 to 3, **characterised in that** the number of elongate elevations (2a, 2b, 2c, 2d, 2') is 4 to 20, preferably 6 to 12, it being preferred that the elongate elevations arranged on each one of the sides of the axis X extend parallel to each other.

5. Back pad according to any one of the preceding claims, **characterised in that** the elongate elevations (2a, 2b, 2c, 2d, 2') have a length in the range of from 1 to 15 cm, preferably 2 to 20 cm.

6. Back pad according to any one of the preceding claims, **characterised in that** the elongate elevations (2a, 2b, 2c, 2d, 2') have a width in the range of from 0.5 to 3 cm, preferably 1 to 2 cm.

7. Back pad according to any one of the preceding claims, **characterised in that** at least two of the elongate elevations (2a, 2b, 2c, 2d, 2') are of different length.

8. Back pad according to any one of the preceding claims, **characterised in that** the elongate elevations (2a, 2b, 2c, 2d, 2') are arranged in a V-shape or Λ-shape, it being preferred that the angle α formed thereby be 90° to 175°, preferably 120° to 160°.

9. Back pad according to any one of the preceding claims, **characterised in that** the elongate elevations (2a, 2b, 2c, 2d, 2') have a height of 2-20 mm, preferably 2-5 mm.

10. Back pad according to any one of the preceding claims, **characterised in that** the height of the elongate elevations (2a, 2b, 2c, 2d, 2'), or of at least some of these elevations, decreases towards the lateral edge (3, 3').

11. Back pad according to any one of the preceding claims, **characterised in that** it is constructed as a lumbosacral back pad and has a first surface region (A), wherein said bulge-like elongate elevations (2a, 2b, 2c, 2d, 2') are provided, and a second surface region (B), which is provided with a plurality of raised knobs (6, 6').

12. Back pad according to claim 11, **characterised in that**, in plan view, the knobs (6, 6') are circular, ellipsoidal, triangular, quadrangular or polygonal, or have an irregular round shape, it being preferred that the diameter be 0.5 cm to 2 cm, preferably 1 cm to 1.5 cm.

13. Back pad according to claim 11 or 12, **characterised in that** the knobs (6, 6') have a height of 0.2 cm to 1 cm.

14. Back pad according to any one of claims 11 to 13, **characterised in that** its plan-view outline has essentially the shape of an isosceles triangle or isosceles trapezium, preferably with rounded corners or/and concave-rounded or convex-rounded sides.

15. Back pad according to any one of the preceding claims, **characterised in that** the plastics material is a foamed plastic, preferably selected from the group comprising polyethylene foam, silicone foam, foamed plastics based on natural or synthetic rubber, and polyurethane flexible foam, with polyurethane flexible foam being especially preferred.

16. Back pad according to any one of the preceding claims, **characterised in that** it is made in one piece.

17. Back pad according to any one of the preceding claims, **characterised in that** on its lower side (20), which is opposite the side (10) that is intended to be in contact with the back, it has one or more fastening means (7), preferably selected from the group of the hook-and-loop fasteners, snap fasteners, loops, eyes and hooks.

18. Back pad according to any one of the preceding claims, **characterised in that** it is enclosed by a cover or bag which covers at least the surface of the side that is intended to be in contact with the back.

## Revendications

1. Coussin d'appui pour le dos (1) pour le massage et la stimulation de la musculature du dos, qui est fabriqué à base d'un matériau plastique élastique et qui présente sur le côté destiné au contact avec le dos une pluralité d'élévations (2a, 2b, 2c, 2d, 2') allongées en forme de renflement, qui sont disposées des deux côtés d'un axe longitudinal (X) du coussin d'appui et sont disposées à une distance de cet axe, de préférence de façon symétrique, et qui sont agencées en direction du bord (3, 3') latéral du coussin d'appui, le tracé de l'axe (X) correspondant au tracé de la colonne vertébrale dans le sens longitudinal, **caractérisé en ce que** au moins certaines des élévations (2a, 2b, 2c, 2d, 2') allongées sont dotées dans leur tracé longitudinal d'épaississements de type noeud ou de bosses (5), de façon à obtenir un profilé de hauteur ondulé.

2. Coussin d'appui pour le dos selon la revendication 1, **caractérisé en ce que** les élévations (2a, 2b, 2c, 2d, 2') allongées sont disposées à une distance de l'axe X tel qu'au moins une zone (4) en forme de bande d'une largeur d'au moins 1,5 cm, de préférence d'au moins 2 cm, reste le long de cet axe, laquelle zone ne présente pas d'élévations.

3. Coussin d'appui pour le dos selon la revendication 2, **caractérisé en ce que** la largeur de la zone (4) en forme de bande est de 1,5 à 10 cm, de préférence de 2 à 5 cm.

4. Coussin d'appui pour le dos selon l'une quelconque des revendications 1 à 3, **caractérisé en ce que** le nombre des élévations (2a, 2b, 2c, 2d, 2') allongées se situe entre 4 et 20, de préférence entre 6 et 12, et dans lequel il est préféré que les élévations allongées disposées sur chaque côté de l'axe X soient de préférence parallèles entre elles.

5. Coussin d'appui pour le dos selon l'une quelconque des revendications précédentes, **caractérisé en ce que** les élévations (2a, 2b, 2c, 2d, 2') allongées présentent une longueur de l'ordre de 1 à 15 cm, de préférence de 2 à 20 cm.

6. Coussin d'appui pour le dos selon l'une quelconque des revendications précédentes, **caractérisé en ce que** les élévations (2a, 2b, 2c, 2d, 2') allongées présentent une largeur de l'ordre de 0,5 à 3 cm, de préférence de 1 à 2 cm.

7. Coussin d'appui pour le dos selon l'une quelconque des revendications précédentes, **caractérisé en ce qu'**au moins deux des élévations (2a, 2b, 2c, 2d, 2') allongées présentent une longueur différente.

8. Coussin d'appui pour le dos selon l'une quelconque des revendications précédentes, **caractérisé en ce que** les élévations (2a, 2b, 2c, 2d, 2') allongées sont disposées en forme de V ou Λ, dans lequel il est préféré que l'angle α ainsi formé soit compris entre 90 et 175°, de préférence entre 120° et 160°.

9. Coussin d'appui pour le dos selon l'une quelconque des revendications précédentes, **caractérisé en ce que** les élévations (2a, 2b, 2c, 2d, 2') allongées présentent une hauteur de 2 à 20 mm, de préférence de 2 à 5 mm.

10. Coussin d'appui pour le dos selon l'une quelconque des revendications précédentes, **caractérisé en ce que** la hauteur des élévations (2a, 2b, 2c, 2d, 2') allongées ou au moins de certaines de ces élévations diminuent en direction du bord (3, 3') latéral.

11. Coussin d'appui pour le dos selon l'une quelconque des revendications précédentes, **caractérisé en ce qu'**il est conçu comme coussin d'appui pour la zone lombaire sacrée et présente une première zone de surface (A), dans laquelle lesdites élévations (2a, 2b, 2c, 2d, 2') allongées en forme de renflement sont placées, ainsi qu'une seconde zone de surface (B), qui est dotée d'une pluralité de noppes (6, 6') surélevées.

12. Coussin d'appui pour le dos selon la revendication 11, **caractérisé en ce que** les noppes (6, 6') ont, en projection horizontale, une forme circulaire, elliptique, triangulaire, quadrangulaire ou polygonale, ou une forme ronde irrégulière, dans lequel il est préféré que le diamètre soit de 0,5 cm à 2 cm, de préférence de 1 cm à 1,5 cm.

13. Coussin d'appui pour le dos selon la revendication 11 ou 12, **caractérisé en ce que** les noppes (6, 6') présentent une hauteur de 0,2 cm à 1 cm.

14. Coussin d'appui pour le dos selon l'une quelconque des revendications 11 à 13, **caractérisé en ce que** sa projection horizontale présente sensiblement la forme d'un triangle équilatérale ou d'un trapèze équilatéral, de préférence avec des angles arrondis et/ou des côtés arrondis de façon concave ou convexe.

15. Coussin d'appui pour le dos selon l'une quelconque des revendications précédentes, **caractérisé en ce que** le matériau plastique est une mousse plastique de préférence choisie dans le groupe comprenant de la mousse de polyéthylène, de la mousse de silicone, des mousses à base de caoutchouc naturel ou de caoutchouc synthétique et de la mousse souple de polyuréthane, dans lequel la mousse souple de polyuréthane est particulièrement préférée.

16. Coussin d'appui pour le dos selon l'une quelconque des revendications précédentes, **caractérisé en ce qu'**il est fabriqué d'une seule pièce.

17. Coussin d'appui pour le dos selon l'une quelconque des revendications précédentes, **caractérisé en ce qu'**il présente sur son côté inférieur (20), qui fait face au côté (10) destiné au contact avec le dos, un ou plusieurs moyens de fixation (7), de préférence sélectionné dans le groupe des bandes auto-aggripantes boutons-pression, boucles, oeillets et crochets.

18. Coussin d'appui pour le dos selon l'une quelconque des revendications précédentes, **caractérisé en ce qu'**il est entouré par une enveloppe ou une poche, laquelle recouvre au moins la surface du côté destiné au contact avec le dos.
